# EUROPEAN PATENT SPECIFICATION

(11) **EP 2 799 237 B1**
(45) Date of publication and mention of the grant of the patent: **30.09.2015**
(21) Application number: 13166521.8
(22) Date of filing: 03.05.2013
(51) Int. Cl.: B32B 37/14, B32B 38/00, B32B 27/12, A61F 13/15

(54) **Method of making an elastic laminate**
Verfahren zur Herstellung eines elastischen Laminats
Procédé de fabrication d'un stratifié élastique

(43) Date of publication of application: 05.11.2014
(73) Proprietor: Mondi Gronau GmbH, 48599 Gronau (DE)
(72) Inventor: Sollmann, Henner, 48599 Gronau (DE); Schönbeck, Marcus, 33775 Versmold (DE)
(74) Representative: Albrecht, Rainer Harald

(56) References cited:
- EP-A1- 1 374 814
- DE-A1-102004 035 649
- US-A1- 2006 254 698

## Description

The invention relates to a method of making an elastic laminate. The invention is directed to a method in which elastic strips are laminated next to one another between nonwoven material webs. The nonwoven material webs are supplied without prestretching and joined to the strips. The laminate thus formed is then stretched transverse to the web direction in regions rendered elastic by the laminated strips, and after elastic relaxation is wound into a roll. Due to the stretching of the laminate, which is also referred to as mechanical activation, the elastic properties of the laminate transverse to the web direction of the material web (CD direction) are improved.

A method having the described features is known from US 2006/254698 or EP 1 686 209, for example. Elastic elements for hygiene products, in particular elastic closure strips for diapers, may be punched from the laminate, the elastic elements each having an elastic center region and less elastic sections at both ends. The inelastic or less elastic end regions are used to fasten hook-and-loop elements, for example hook tapes, and to attach the elastic element to inelastic surfaces of a diaper. The laminate is manufactured as a wide web having a plurality of laminated elastic strips. The closure strips necessary for diaper manufacture may be punched from the resulting multiuse material.

A method of making an elastic nonwoven laminate is known from EP 1 844 928 in which an elastic film is preactivated by stretching, followed by elastic relaxation, then stretched again, and in the stretched state is laminated onto a nonwoven web. The stretching forces to which the elastic film is subjected during the lamination onto the nonwoven web may be reduced by preactivating the elastic film. The bond strength between the layers of the laminate may also be improved by preactivating the film. However, the lamination procedure using an elastic film that is stretched during the lamination process is complicated and not technically practical when a number of elastic strips oriented parallel to one another must be laminated between two nonwoven material webs, and a laminate having elastic and inelastic regions is to be made.

In a method known from US 2006/0003656 for making an elastic nonwoven laminate, an elastic film is stretched transverse to the web direction, and after elastic relaxation is laminated onto a nonwoven web that is stretched in the web direction and in the stretched state is bonded to the elastic film. As the result of stretching the nonwoven web, the material width of the nonwoven is reduced, and folds are formed in the nonwoven web that impart transverse elasticity to the laminate (CD direction). However, it is not possible to use the described method to make a laminate that has adjacent elastic and inelastic sections in the web longitudinal direction.

Against this background, the object of the invention is to provide a method of making a laminate that has nonwoven cover layers and elastic strips between the cover layers, and that may be stretched with a low force over a large stretching area only in the areas in which the strips are laminated.

The subject matter of the invention and the achievement of this object are provided in a method according to claim 1.

In the method according to the invention, an elastic film is stretched transverse to the web direction and, after elastic relaxation, is cut into strips. The strips are laminated next to one another between nonwoven material webs supplied without prestretching and joined to the strips. Last, the laminate thus formed is stretched transverse to the web direction in regions rendered elastic by the laminated strips and, after elastic relaxation, is wound into a roll. Closure elements may be punched from the material that have an elastic center region and less elastic end sections at both ends.

Stretching the elastic film mechanically preactivates a component of the laminate and results in an improvement in the stretching behavior of the laminate. The preactivation of the elastic film has a positive effect on the stretching force profile of the laminate and contributes to the laminate being easily stretchable over a large stretching area while greatly increasing the stretching resistance for a yield strength determined by the preactivation of the elastic film, the stretching resistance being readily determined as the yield strength upon subsequent use of the laminate. In addition, the elastic relaxation behavior of the laminate after strain relief may be improved by using a preactivated elastic film. However, the preactivation of the elastic film does not replace the mechanical activation of the laminate, but instead cooperates with it synergistically. In the preactivation of the elastic film, the film is preferably stretched essentially uniformly over its entire width. In contrast, the stretching of the laminate for mechanical activation is locally limited to the regions of the laminate that are already elastic due to the laminated strips that are preactivated according to the invention. Due to the stretching of the laminate, fibers of the nonwoven layers are irreversibly stretched in the elastic regions of the laminate, and bonding of the nonwoven in the elastic regions is reduced due to fiber tears and fiber rearrangements. This is accompanied by a renewed mechanical effect on the material of the elastic strips, as well as a mechanical effect on localized bonds between the film surface and adjacent fibers. Areas of the laminate between the elastic regions are not altered by stretching the laminate and retain the properties of the nonwoven.

For purposes of the preactivation, the elastic film is preferably stretched transversely by 100% to 500%. These numerical values refer to the change in length of the film transverse to the web longitudinal direction relative to the starting width of the film. The value of 100% means that the film in the stretched state has a width that is twice the starting width of the film. The stretching is not fully reversible. As the result of inelastic portions of the film, after its elastic relaxation the film has a slightly greater width than prior to the stretching. The width subsequent to the elastic relaxation may be approximately 10% to 30% greater than the starting width of the elastic film prior to transverse stretching.

For preactivation of the elastic film, i.e. for the transverse stretching of the elastic film prior to its further processing, a stretching roller system composed of intermeshing profile rollers is preferably used. The profile rollers may in particular be composed of multiple disks that are combined into packets, the disks preferably being arranged equidistantly for uniform stretching transversely of the web.

After preactivation, the elastic film is cut into strips. The strips are guided over deflectors and may be supplied as parallel strips to a lamination unit where the strips are laminated between nonwoven webs that are supplied on the top and bottom faces. The elastic strips are advantageously spaced from one another. The spacing between the strips may be set by positioning the deflectors. The nonwoven webs are directly joined together in the gaps between the elastic strips. It is also within the scope of the invention that the areas between the elastic strips are reinforced by colaminated reinforcing strips. Elastic and inelastic regions may thus be formed in the laminate.

For the mechanical activation, the laminate may be guided through a nip between two profile rollers, each including at least two disk packets, having a plurality of disks, situated on an axis. The laminate is stretched in places by intermeshing disk packets of the two profile rollers. In roller sections between the disk packets, the profile rollers form a gap, through which the laminate is guided essentially without transverse stretching.

Relative to the overall width of the laminated strips, the maximum transverse stretching of the laminate for the mechanical activation corresponds to the value by which the elastic film is stretched for purposes of preactivation. In other words, in the area of the laminated strips, the maximum stretching of the laminate is as great as that of the elastic film during its preactivation. The transverse stretching of the laminate for the mechanical activation (relative to the overall width of the laminated strips) is preferably 50% to 90% of the value by which the film is stretched for purposes of preactivation.

A film composed of a polyolefin elastomer is preferably used as elastic film. The preactivation of the elastic film is particularly effective when an elastic film based on polyolefin elastomers is used.

A single-layer film or a multilayer film having an elastomeric core layer composed of styrene-isoprene-styrene (SIS) block copolymers, styrene-butadiene-styrene (SBS) block copolymers, styrene-ethylene/butylene-styrene (SEBS) block copolymers, polyurethanes, ethylene copolymers, or polyether block amides may also be used as elastic film.

The nonwoven from which the cover layers of the laminate are made has fibers made of stretchable polymers that have only slight elasticity compared to the polymer of the elastic film. The nonwoven may be composed of melt-blown fibers, staple fibers, or continuous fibers, the fibrous web formed from the fibers being mechanically, thermally, or chemically bonded. In particular, spunlace nonwovens may also be used as cover layers.

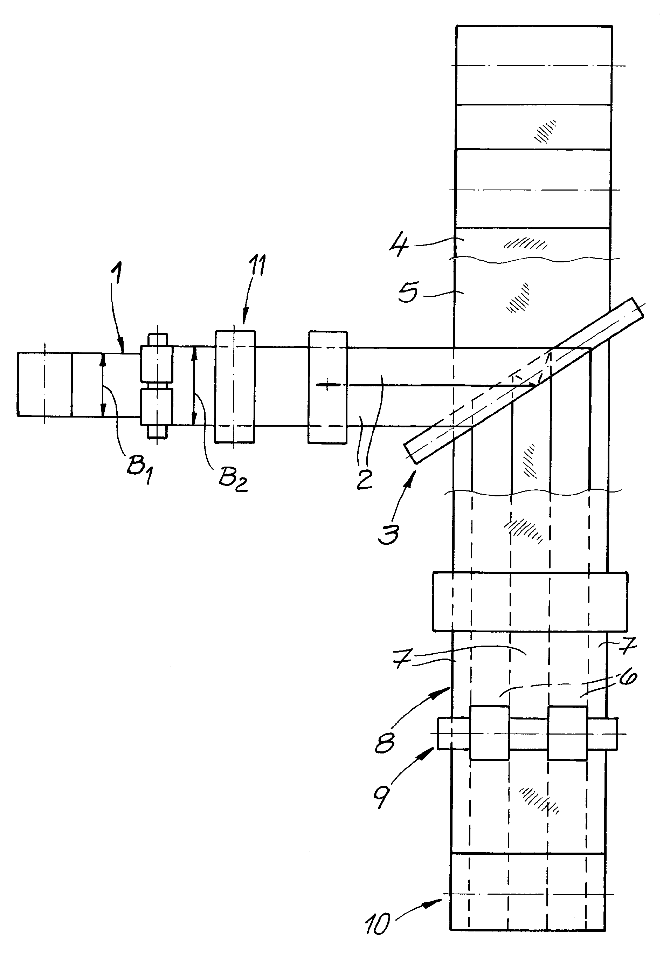

The invention is explained below with reference to a single embodiment. The sole figure schematically shows a method of making an elastic laminate.

In the method shown in the figure, an elastic film 1 is stretched transverse to a web direction in a preactivation station 11, and after elastic relaxation is cut into strips 2. The strips 2 are guided over deflectors 3, and as parallel strips are laminated next to one another between two nonwoven material webs 4 and 5. The material webs 4 and 5 are guided above and beneath the strips 2 without prestretching, and are adhesively or thermally bonded to the strips 2. The view clearly shows that the elastic strips 2 are laminated at a spacing from one another between the cover layers, and that the nonwoven cover layers 4 and 5 are directly joined together in the gaps between the elastic strips 2. Elastic regions 6 and inelastic regions 7 are thus formed in the laminate 8. The laminate is supplied to an activation unit 9, in which the laminate 8 is stretched transverse to the web direction in the regions 6 rendered elastic by to the laminated strips 2. After elastic relaxation, the laminate 8 is wound into a roll 10.

The elastic film 1 is stretched transverse to the web direction by more than 50% in the preactivation station 11. The stretching occurs essentially uniformly over the entire width of the film 1. The elastic film is preferably stretched by 100% to 300% relative to its starting width of the elastic film, stretching to 500% also being possible. After the elastic relaxation, the elastic film 1 has a width B₂ that is 10% to 30% greater than the starting width B₁ of the elastic film. The stretching of the elastic film 1 constitutes a preactivation that has an advantageous effect on the elongation values of the laminate 8. A stretching roller system composed of intermeshing profile rollers may be used to preactivate the elastic film 1.

A single-layer elastomer film or a multilayer film having an elastomeric core layer composed of styrene-isoprene-styrene block copolymers, styrene-butadiene-styrene block copolymers, styrene-ethylene/butylene-styrene block copolymers, polyurethanes, ethylene copolymers, or polyether block amides may be used as elastic film. An elastic blown film composed of a polyolefin elastomer is particularly preferably used.

The stretching of the laminate 8 is limited to the regions of the laminate 8 that have been made elastic by the laminated and preactivated strips 2. For this purpose, the laminate 8 is guided through a nip between two profile rollers that include at least two packets of a plurality of disks mounted on an axle. The laminate is stretched in places by the intermeshing disk packets of the two profile rollers. As a result of the stretching, textile structures of the cover layers are altered in the elastic regions 6 of the laminate, and the elongation properties of the laminate 8 in the CD direction, i.e. transverse to the web longitudinal direction, are improved. Relative to the overall width of the laminated strips 2, the maximum transverse stretching of the laminate during stretching corresponds to the value by which the elastic film 1 is stretched during preactivation. The transverse stretching of the laminate 8 relative to the overall width of the laminated strips is preferably 50% to 90% of the value by which the elastic film 1 is stretched during preactivation. Next to the disk packets, the profiles have roller sections in which the laminate is not subjected to transverse stretching. These sections define a nip through which the laminate 8 is guided essentially without transverse stretching.

## Claims

1. A method of making an elastic laminate, wherein
a) an elastic film (1) is stretched transverse to a web direction and, after elastic relaxation, is cut into strips (2),
b) the strips (2) are laminated next to one another between nonwoven material webs (4, 5) supplied without prestretching and joined to the strips (2),
c) the laminate (8) thus formed is stretched transverse to the web direction in regions (6) rendered elastic by the strips (2) and, after elastic relaxation, is wound into a roll (10).

2. The method according to claim 1, **characterized in that** in method step a) the elastic film (1) is stretched essentially uniformly over its entire width.

3. The method according to claim 1 or 2, **characterized in that** in method step a) the elastic film (1) is stretched transversely by 100% to 500%.

4. The method according to one of claims 1 through 3, **characterized in that** a stretching roller system composed of intermeshing profile rollers is used for stretching the elastic film (1).

5. The method according to one of claims 1 through 4, **characterized in that** the strips (2) are laminated at a distance from one another between the nonwoven material webs (4, 5) so as to form elastic regions (6) and inelastic regions (7) in the laminate (8).

6. The method according to one of claims 1 through 5, **characterized in that** the strips (2) are guided over deflectors (3) and are supplied as parallel strips to a lamination unit in which the strips (2) are laminated between nonwoven webs (4, 5) that are supplied on top and bottom faces.

7. The method according to one of claims 1 through 6, **characterized in that** in method step c) the laminate (8) is guided through a nip between two profile rollers that each include at least two packets each having a plurality of disks mounted on an axle, the laminate being locally stretched by the intermeshing disk packets of the two profile rollers and guided through a nip next to the disk packets essentially without transverse stretching.

8. The method according to one of claims 1 through 7, **characterized in that**, relative to the overall width of the laminated strips (2), the maximum transverse stretching of the laminate (8) in method step c) corresponds to a value by which the elastic film (1) is stretched in method step a).

9. The method according to claim 8, **characterized in that**, relative to the overall width of the laminated strips, the transverse stretching of the laminate in method step c) corresponds to 50% to 90% of the value by which the elastic film (1) is stretched in method step a).

10. The method according to one of claims 1 through 9, **characterized in that** a film composed of a polyolefin elastomer is used as the elastic film (1).

11. The method according to one of claims 1 through 9, **characterized in that** a single-layer film or a multilayer film having an elastomeric core layer composed of styrene-isoprene-styrene block copolymers, styrene-butadiene-styrene block copolymers, styrene-ethylene/butylene-styrene block copolymers, polyurethanes, ethylene copolymers, or polyether block amides may be used as elastic film (1).

## Patentansprüche

1. Verfahren zur Herstellung eines elastischen Laminats, wobei:
a) eine elastische Folie (1) quer zu einer Bahnrichtung gestreckt und nach der elastischen Entspannung in Streifen (2) geschnitten wird,
b) die Streifen (2) nebeneinander zwischen Vliesmaterialbahnen (4, 5) laminiert werden, die ohne Vorstreckung zugeführt und mit den Streifen (2) verbunden werden,
c) das so gebildete Laminat (8) quer zur Bahnrichtung in Bereichen (6) gestreckt wird, denen durch die Streifen (2) Elastizität verliehen wird, und das Laminat nach der elastischen Entspannung auf eine Rolle (10) gewickelt wird.

2. Verfahren nach Anspruch 1, **dadurch gekennzeichnet, dass** die elastische Folie (1) im Verfahrensschritt a) über ihre gesamte Breite im Wesentlichen gleichmäßig gestreckt wird.

3. Verfahren nach Anspruch 1 oder 2, **dadurch gekennzeichnet, dass** die elastische Folie (1) im Verfahrensschritt a) um 100 bis 500 % quergestreckt wird.

4. Verfahren nach einem der Ansprüche 1 bis 3, **dadurch gekennzeichnet, dass** zum Strecken der elastischen Folie (1) ein Streckwalzensystem verwendet wird, das aus ineinandergreifenden Profilwalzen besteht.

5. Verfahren nach einem der Ansprüche 1 bis 4, **dadurch gekennzeichnet, dass** die Streifen (2) in einem Abstand zueinander derart zwischen die Vliesmaterialbahnen (4, 5) laminiert werden, dass im Laminat (8) elastische Bereiche (6) und nichtelastische Bereiche (7) gebildet werden.

6. Verfahren nach einem der Ansprüche 1 bis 5, **dadurch gekennzeichnet, dass** die Streifen (2) über Ablenkelemente (3) geleitet und als parallele Streifen zu einer Laminiereinheit geführt werden, in der die Streifen (2) zwischen die Vliesbahnen (4, 5) laminiert werden, die an der Ober- und der Unterseite zugeführt werden.

7. Verfahren nach einem der Ansprüche 1 bis 6, **dadurch gekennzeichnet, dass** das Laminat (8) im Verfahrensschritt c) durch einen Spalt zwischen zwei Profilwalzen geleitet wird, die jeweils mindestens zwei Pakete mit jeweils mehreren auf einer Achse montierten Scheiben aufweisen, wobei das Laminat durch die ineinandergreifenden Scheibenpakete der zwei Profilwalzen örtlich gestreckt und im Wesentlichen ohne Querstreckung durch einen Spalt neben den Scheibenpaketen geleitet wird.

8. Verfahren nach einem der Ansprüche 1 bis 7, **dadurch gekennzeichnet, dass** die maximale Querstreckung des Laminats (8) im Verfahrensschritt c) im Verhältnis zur Gesamtbreite der laminierten Streifen (2) einem Wert entspricht, um den die elastische Folie (1) im Verfahrensschritt a) gestreckt wird.

9. Verfahren nach Anspruch 8, **dadurch gekennzeichnet, dass** die Querstreckung des Laminats im Verfahrensschritt c) im Verhältnis zur Gesamtbreite der laminierten Streifen 50 bis 90 % des Wertes entspricht, um den die elastische Folie (1) im Verfahrensschritt a) gestreckt wird.

10. Verfahren nach einem der Ansprüche 1 bis 9, **dadurch gekennzeichnet, dass** eine Folie als elastische Folie (1) verwendet wird, die aus einem Polyolefinpolymer besteht.

11. Verfahren nach einem der Ansprüche 1 bis 9, **dadurch gekennzeichnet, dass** als elastische Folie (1) eine einschichtige Folie oder eine mehrschichtige Folie verwendet werden kann, die eine Elastomerkernschicht aufweist, die aus Styrol-Isopren-Styrol-Blockcopolymeren, Styrol-Butadien-Styrol-Blockcopolymeren, Styrol-Ethylen/Butylen-Styrol-Blockcopolymeren, Polyurethanen, Ethylen-Copolymeren oder Polyether-Blockamiden besteht.

## Revendications

1. Procédé de fabrication d'un laminé élastique, dans lequel
a) un film (1) élastique est étiré transversalement dans un sens de la nappe puis coupé en bandes (2) après détente élastique,
b) les bandes (2) sont laminées ensuite rapprochées les unes des autres, des bandes de matériau non tissé (4, 5) étant fournies sans pré-étirage et assemblées aux bandes (2),
c) le laminé (8) ainsi formé est étiré transversalement au sens de la nappe dans des zones (6) rendues élastiques par les bandes (2) puis enroulé en rouleau (10) après détente élastique.

2. Procédé selon la revendication 1, **caractérisé en ce que**, dans l'étape a) du procédé, le film élastique (1) est étiré sensiblement uniformément sur toute sa largeur.

3. Procédé selon la revendication 1 ou 2, **caractérisé en ce que**, dans l'étape a) du procédé, le film élastique (1) est étiré transversalement à raison de 100 % à 500 %.

4. Procédé selon l'une quelconque des revendications 1 à 3, **caractérisé en ce qu'**un système de rouleau d'étirement composé de rouleaux à profils imbriqués est utilisé pour étirer le film élastique (1).

5. Procédé selon l'une quelconque des revendications 1 à 4, **caractérisé en ce que** les bandes (2) sont laminées à distance les unes des autres entre les nappes de matériau non tissé (4, 5) de manière à former des zones élastiques (6) et des zones inélastiques (7) dans le laminé (8).

6. Procédé selon une des revendications 1 à 5, **caractérisé en ce que** les bandes (2) sont guidées via des déflecteurs (3) et sont fournies sous forme de bandes parallèles à une unité de laminage dans laquelle les bandes (2) sont laminées entre des nappes de matériau non tissé (4, 5) qui sont posées sur les faces supérieure et inférieure.

7. Procédé selon une des revendications 1 à 6, **caractérisé en ce que**, dans l'étape c) du procédé, le laminé (8) est guidé dans un intervalle entre deux rouleaux à profils qui comprennent chacun au moins deux ensembles dotés chacun d'une pluralité de disques montés sur un axe, le laminé étant étiré localement par les ensembles de disques imbriqués des deux rouleaux à profils et guidé dans un intervalle près des ensembles de disques sensiblement sans étirement transversal.

8. Procédé selon une des revendications 1 à 7, **caractérisé en ce que**, par rapport à la largeur totale des bandes laminées (2), l'étirement transversal maximal du laminé (8) dans l'étape c) du procédé correspond à une valeur à raison de laquelle le film élastique (1) est étiré dans l'étape a) du procédé.

9. Procédé selon la revendication 8, **caractérisé en ce que**, par rapport à la largeur totale des bandes laminées, l'étirement transversal du laminé dans l'étape c) du procédé correspond à 50 % à 90 % de la valeur à raison de laquelle le film élastique (1) est étiré dans l'étape a) du procédé.

10. Procédé selon une des revendications 1 à 9, **caractérisé en ce qu'**un film composé d'un élastomère de polyoléfine est utilisé comme film élastique (1).

11. Procédé selon une des revendications 1 à 9, **caractérisé en ce qu'**un film monocouche ou un film multicouche dont une couche de noyau élastomérique est composée de copolymères à blocs de styrène-isoprène-styrène, copolymères à blocs de styrène-butadiène-styrène, copolymères à blocs de styrène-éthylène/butylène-styrène, polyuréthanes, copolymères d'éthylène ou d'amides à blocs de polyéther peut être utilisé comme film élastique (1).
